# FASCICULE DE BREVET EUROPEEN

(11) **EP 0 840 648 B1**
(45) Date de publication et mention de la délivrance du brevet: **05.09.2001**
(21) Numéro de dépôt: 97925131.1
(22) Date de dépôt: 23.05.1997
(51) Int. Cl.: B03C 1/01, H01F 1/37, H01F 1/44, G01N 33/543

(54) **PARTICULES SUPERPARAMAGNETIQUES ET MONODISPERSES**
SUPERPARAMAGNETISCHE UND MONODISPERGIERTE TEILCHEN
SUPERPARAMAGNETIC MONODISPERSED PARTICLES

(30) Priorité: 24.05.1996 FR 9606765
(43) Date de publication de la demande: 13.05.1998
(73) Titulaire: BIO MERIEUX, 69280 Marcy l'Etoile (FR)
(72) Inventeur: ELAISSARI, Abdelhamid, F-69007 Lyon (FR); PICHOT, Christian, F-69960 Corbas (FR); MANDRAND, Bernard, F-69100 Villeurbanne (FR); SAUZEDDE, Florence, F-69007 Lyon (FR)
(74) Mandataire: Guerre, Dominique
(86) Numéro de dépôt international: FR9700912
(87) Numéro de publication internationale: WO9745202

(56) Documents cités:
- EP-A- 0 585 868
- WO-A-91/09141
- A.KONDO ET AL: "Development and application of thermo-sensitive magnetic immunomicrospheres for antibody purification" APPLIED MICROBIOLOGY AND BIOTECHNOLOGY, vol. 41, 1994, BERLIN DE, pages 99-105, XP000613881 cité dans la demande

## Description

La présente invention concerne des particules superparamagnétiques, monodisperses, leur procédé de préparation ainsi que leurs utilisations notamment en biologie dans l'isolement de molécules biologiques.

L'état de la technique révèle des particules superparamagnétiques et monodisperses. A titre d'illustration, les documents **EP-0 106 873** et **EP-0 446 260** décrivent des particules superparamagnétiques et monodisperses comprenant un noyau poreux à base de copolymère polystyrène/divinylbenzène dans lequel sont incorporés des grains d'oxyde de fer magnétique, et une couche externe fonctionnalisée susceptible d'interagir avec des sondes d'acides nucléiques.

Selon le procédé de préparation des particules décrites dans ces documents, les oxydes de fer magnétiques sont incorporés par précipitation des sels correspondants, ce qui limite la proportion de charge magnétique incorporée, et ne permet d'obtenir la charge magnétique qu'en une monocouche.

Le document **EP-0 585 868** décrit des particules magnétiques constituées par un noyau à base d'un premier polymère et d'une couche magnétique recouvrant le noyau constituée d'un second polymère dans lequel est distribué le matériau magnétique à base de ferrite, et capable d'interagir avec un antigène ou un anticorps, le matériau magnétique étant déposé par précipitation des sels de fer.

Le matériau magnétique incorporé est directement exposé aux traitements ultérieurs des particules et il s'ensuit une perte de la charge au cours de l'utilisation des particules, ce qui peut entraîner des problèmes notamment d'inhibition enzymatique et de dénaturation d'entités biologiques.

Selon l'invention, on apporte des particules qui sont superparamagnétiques, qui ont une charge magnétique distribuée de manière très homogène, dont la proportion peut varier entre 1 à 80 %, en particulier de 25 à 80 % en poids par rapport au(x) polymère(s) constituant les particules. La présente invention permet d'atteindre des proportions de charge magnétique incorporée élevées, en particulier car le procédé employé permet de répartir la charge magnétique sous forme de multicouches. Il en résulte un avantage considérable à savoir la possibilité de séparer efficacement de l'échantillon, les particules de l'invention, sans avoir recours à l'action combinée d'une autre technique de séparation, telle que la floculation.

La charge magnétique se présente sous la forme de nanoparticules qui sont incorporées dans les particules de manière substantiellement irréversible, c'est-à-dire sans perte par relargage, quels que soient les traitements ultérieurs qui leur sont appliqués, notamment dans l'échantillon, à savoir rinçages, variations de températures, de pH...

Les propriétés des particules de l'invention résultent de leur structure et composition particulières et plus précisément à la présence d'un polymère thermosensible constitutif au moins pour supporter le matériau magnétique.

Selon l'article de A. KONDO, (A. KONDO, H. KAMURA, et K. HIGASHITANI (1994) Appl. Microbiol. Biotechnol., 41, 99-105) on connaît un procédé d'obtention de particules magnétiques, comprenant un noyau à base d'un premier polymère consistant en un polystyrène et dans lequel est distribué un matériau magnétique, et une couche hydrophile recouvrant le noyau, à base d'un polymère thermosensible consistant en du poly(N-isoproprylacrylamide). Le procédé décrit comprend les étapes suivantes :
- selon une première étape pour l'obtention du noyau magnétique, on met en contact le matériau magnétique avec du styrène en présence d'un amorceur de polymérisation, puis
- selon une seconde étape pour l'obtention de la couche hydrophile, on met en contact le noyau obtenu avec du N-isopropylacrylamide et de l'acide méthacrylique, en présence de l'amorceur de polymérisation précédent.

Sur les particules ainsi obtenues, on fixe de la sérumalbumine bovine pour ensuite procéder à l'isolement d'anticorps dirigés contre la sérum albumine bovine, présents dans un échantillon.

L'inconvénient de ces particules survient à l'étape de leur séparation : ces particules incorporent une faible proportion de charge magnétique et sont en outre de tailles très variables, limitant considérablement l'efficacité d'un champ magnétique appliqué pour séparer les particules. Ainsi pour assurer une séparation aussi efficiente que possible de ces particules dans l'échantillon, les auteurs ont recours à une thermoflocculation selon laquelle on augmente la température de l'échantillon, qui intervient pour compléter l'action d'un champ magnétique.

La nécessité d'une technique de séparation complémentaire résulte des particules obtenues qui présentent les inconvénients suivants :
- proportions de charge magnétique incorporée faibles,
- répartition non homogène de la charge magnétique, et
- obtention de particules non monodisperses.

Les particules de l'invention sont destinées à l'isolement de molécules biologiques, essentiellement par l'application d'un champ magnétique, indépendamment de toutes variations de température, pH, force ionique.

Les particules superparamagnétiques et monodisperses de l'invention ont une taille prédéterminée comprise entre 0,1 et 10 µm et comprennent :
- un noyau à base d'un premier polymère,
- une couche interne dite couche magnétique, recouvrant le noyau, à base d'un second polymère et dans laquelle est distribué un matériau magnétique, et
- une couche externe, dite couche d'encapsulation, éventuellement fonctionnalisée, recouvrant la couche magnétique, à base d'un troisième polymère et susceptible d'interagir avec au moins une molécule biologique,
au moins le second polymère étant thermosensible et présentant une température critique inférieure de solubilité (LCST) prédéterminée comprise entre 15 et 65°C, et de préférence entre 25 et 50°C.

Avantageusement, le second polymère est obtenu par polymérisation de (1) un monomère hydrosoluble, d'acrylamide ou d'un dérivé d'acrylamide, tel que le N-isopropylacrylamide (NIPAM), (2) au moins un agent de réticulation, tel que le N,N-méthylène bisacrylamide, et (3) au moins un monomère fonctionnel, cationique et hydrosoluble, différent du monomère (1), tel que chlorure de 2-aminoéthyl-méthacrylate. Un second polymère préféré est le PNIPAM [poly-(N-isopropylacrylamide)].

Le premier polymère peut être identique au second polymère ou différent du second polymère, dans ce dernier cas le premier polymère sera de préférence un polymère à caractère hydrophobe, et en particulier un polystyrène ou un polyméthacrylate de méthyle.

Le troisième polymère est un polymère compatible avec le second polymère et est choisi parmi les polymères hydrophiles, en particulier les dérivés acrylamides et de préférence le PNIPAM. Lorsque ce troisième polymère est fonctionnalisé, il porte un ou plusieurs groupes fonctionnels choisis parmi les fonctions carboxylique, aldéhydique, thiol et amine.

Un second objet de l'invention est un procédé d'obtention de particules telles que définies précédemment, qui comprend les étapes suivantes :
- selon une étape (a) dite étape d'obtention du premier polymère, on obtient par polymérisation du ou des monomères appropriés, le premier polymère,
- selon une étape (b) dite étape d'obtention du second polymère, on obtient un sol du second polymère, par polymérisation en phase aqueuse de (1) un monomère hydrosoluble, d'acrylamide ou d'un dérivé d'acrylamide, (2) au moins un agent de réticulation et (3) au moins un monomère fonctionnel, cationique et hydrosoluble, différent du monomère (1),
- selon une étape (c) dite d'adsorption du matériau magnétique, on met en contact le matériau magnétique avec les premier et second polymères, à une température inférieure à la LCST du second polymère,
- selon une étape (d) dite d'obtention de la couche magnétique, on porte le mélange réactionnel obtenu selon (c) à une température supérieure à la LCST du second polymère,
- selon une étape (e) dite d'encapsulation, on met en contact, en phase aqueuse, le mélange obtenu selon (d) avec le ou les monomères appropriés pour l'obtention par polymérisation du troisième polymère.

Les étapes (a) et (b) sont, selon une variante du procédé, effectuées simultanément, en particulier mais de manière non limitative quand le premier polymère est identique au second polymère.

Pour l'étape (b) et éventuellement l'étape (a) les réactifs de polymérisation sont de préférence sélectionnés comme suit :
- le monomère (1) est de préférence choisi parmi les N-alkylacrylamides et les N,N-dialkylacrylamides, en particulier parmi le N-isopropylacrylamide, le N-éthylméthacrylamide, N-n-propylacrylamide, le N-n-propylméthacrylamide, N-isopropylméthacrylamide, le N-cyclopropylacrylamide, le N,N-diéthylacrylamide, le N-méthyl-N-isopropylacrylamide, le N-méthyl-N-n-propylacrylamide, le monomère (1) étant de préférence le N-isopropylacrylamide (NIPAM),
- le ou les monomères fonctionnels (3) sont choisis parmi les dérivés acryliques et méthacryliques, le chlorure de 2-aminoéthyl-méthacrylate (AEM), les dérivés de N-vinyl-pyridine, les dérivés de trialkylammonium et les dérivés de chlorure d'isothiouronium, et éventuellement
- l'agent de réticulation (2) est hydrosoluble et est choisi parmi le N,N-méthylène bisacrylamide (MBA), l'éthylène glycol diméthacrylate.

Pour l'étape (c), l'adsorption du matériau magnétique sur le second polymère résulte d'interactions électrostatiques entre des particules de charges opposées. Le milieu réactionnel pour l'adsorption est une phase aqueuse dont les paramètres force ionique et pH sont contrôlés.

L'invention concerne en outre les applications des particules définies ci-dessus. Ainsi les particules sont notamment utilisables pour capturer puis séparer, dans un échantillon liquide, au moins une molécule biologique, notamment choisie parmi les protéines, les anticorps, les fragments d'anticorps, les antigènes, les polypeptides, les enzymes, les haptènes, les acides nucléiques et les fragments d'acides nucléiques. La ou les molécules biologiques sont fixées sur les particules par adsorption ou par liaison covalente, directement ou indirectement, et dans ce dernier cas, par l'intermédiaire d'un ligand par exemple.

Des exemples d'utilisations particulières des particules de l'invention sont les suivantes :
- utilisation comme traceur après concentration magnétique sur une phase solide :
   dans ce cas, la particule est comptée après balayage de la surface par une pointe de microscope de force atomique ou après observation microscopique directe, ou avec une caméra ; les particules magnétiques peuvent être détectées du fait de leur charge métallique et mesurées avec un magnétomètre ou tout système de type lecteur de carte de crédit ; pour faciliter la concentration des particules sur la surface, un aimant permanent ou un électroaimant peut être disposé au-dessous ou au-dessus de la surface utilisée pour la détection ; la concentration magnétique sera préférentiellement effectuée si l'on utilise un nombre limité de particules, par exemple la quantité suffisante pour recouvrir une à dix fois la surface si le processus est statique et une quantité éventuellement plus importante si la concentration est dynamique par circulation contrôlée de liquide sur la surface réactive ;
- utilisations des particules couplées à un ligand biologique approprié dans un protocole réactionnel d'agglutination ; la taille des particules peut être suivie directement dans le milieu ou après attraction magnétique ;
- utilisation des particules pour vectoriser des réactifs dans un dispositif de type capillaire, un jeu d'électroaimants permettant le déplacement des particules ;
- utilisation des particules pour créer des voies préférentielles et/ou obstruer des canaux de distribution de liquide ;
- utilisation des particules pour transporter jusqu'à leurs cibles des substances thérapeutiques :
le principe actif est adsorbé ou transitoirement couplé par covalence sur la surface de la particule, un champ magnétique approprié est appliqué pour entraîner le déplacement de l'ensemble particule-substance thérapeutique.

Un autre objet de l'invention est un procédé pour isoler, dans un échantillon liquide, au moins une molécule biologique, selon lequel :
- on dispose de particules selon l'invention,
- on met en contact ledit échantillon avec lesdites particules, par incubation,
- on applique un champ magnétique au mélange obtenu,
- on sépare les particules de l'échantillon.

Bien entendu, la séparation des particules qui fait l'objet de ce dernier procédé est différente de la séparation des molécules biologiques comprise dans la notion d'isolement. Il s'agit dans le premier cas de séparer de l'échantillon liquide, les particules sur lesquelles est fixée la ou les molécules biologiques, par action d'un champ magnétique.

Enfin un dernier objet de l'invention est un réactif pour l'isolement de molécules biologiques comprenant une dispersion en milieu aqueux de particules telles que définies précédemment.

Avant de décrire plus en détails la présente invention, certains termes employés dans la description sont définis.

Par particules superparamagnétiques, on entend des particules contenant des particules d'un matériau magnétique, garantissant, après suppression du champ magnétique, l'absence de toute aimantation rémanente.

Par particules monodisperses, on comprend des particules ayant sensiblement la même taille, et plus précisément dont la taille varie de 5 % au plus par rapport à une taille moyenne donnée et choisie.

L'expression "isoler une molécule biologique" selon l'invention, comprend la séparation, la détection, d'une molécule biologique, l'enrichissement d'une fraction en une molécule biologique, selon une méthode d'isolement spécifique ou aspécifique, de manière qualitative et/ou quantitative, de manière directe ou indirecte par exemple par l'intermédiaire d'un ligand fixé sur les particules.

### EXEMPLE 1 : PREPARATION DU PREMIER ET DU SECOND POLYMERES

### 1) Le premier et le second polymères sont différents, le premier est un polystyrène, le second est le PNIPAM

Les préparations détaillées ci-après ont utilisées la polymérisation radicalaire en milieu hétérogène, à partir des réactifs de départ suivants :
- pour le premier polymère : le monomère est le styrène (St) (Janssen),
- pour le second polymère :
   le monomère (1) est le N-isopropylacrylamide (NIPAM) (Kodak),
   l'agent de réticulation est le N-N méthylène bisacrylamide (MBA) (Aldrich),
   le monomère (3) fonctionnel est le chlorure de 2-aminoéthyl méthacrylate (AEM) (Kodak),
   l'amorceur de polymérisation est le chlorure de 2,2'-azobis amidino propane (V50) (Wako), et du NaCl a été utilisé pour ajuster la force ionique.

### 1.1) Polymérisation en réacteur fermé (batch)

Tous les monomères précités sont introduits dans le réacteur avant le début de la réaction de polymérisation avec les autres réactifs et sans ajout ultérieur. Cette méthode s'avère très efficace pour la copolymérisation d'un mélange de monomères hydrophobes et hydrophiles, car le monomère hydrophobe (St) forme principalement le noyau et le monomère hydrophile (NIPAM) forme la couche recouvrant le noyau, si la polymérisation a lieu dans la phase aqueuse.

La synthèse est effectuée dans un réacteur de 250 ml sous agitation constante à 200 tours/minutes et sous atmosphère inerte d'azote. L'eau utilisée, bouillie et dégazée sous azote pendant deux heures, est introduite dans le réacteur thermostaté à 70°C et laissée sous un léger courant d'azote pendant 15 minutes, afin d'éliminer toutes traces d'oxygène. Les monomères (St, NIPAM) sont introduits et dégazés pendant encore 15 minutes avant d'ajouter l'amorceur V50.

Formulation du mélange réactionnel :

| Réactifs | Masse |
|---|---|
| Eau | 200 ml |
| St | 18 g |
| NIPAM | 2,06 g |
| V50 | 0,2053 g |

Caractéristiques de la dispersion colloïdale obtenue:

| | |
|---|---|
| (a) diamètre à 20°C | 376 nm |
| (b) diamètre à 50°C | 330 nm |
| (c) diamètre par MET | 326 nm |
| (d) densité de charge | 10 mmol/g |

| | |
|---|---|
| (a) diamètre mesuré par diffusion dynamique de la lumière à 20°C | |
| (b) diamètre mesuré par diffusion dynamique de la lumière à 50°C | |
| (c) diamètre mesuré en microscopie électronique à transmission | |
| (d) densité de charge exprimée en mmole(amine)/g de polymère. | |

### 1.2) Polymérisation sur semence

Cette méthode consiste à introduire le ou les monomères (1) et/ou (3) dans un réacteur contenant la dispersion colloïdale 1.1) déjà constituée et parfaitement caractérisée, en présence du réticulant MBA. Le ou les monomères (1) et/ou (3) peuvent être additionnés sur la semence, en une seule étape ou en semicontinu.

La réaction de polymérisation est faite dans un réacteur de 100 ml, à une température de 70°C, sous une agitation de 200 tours/minutes. La durée de la réaction de polymérisation est de 19 heures.

Formulation du mélange réactionnel identifié sous la référence (PS131/132) :

| Réactifs | Masse (g) |
|---|---|
| Polymère selon 1.1 | 1,26 |
| NIPAM | 0,77 |
| MBA | 0,06 |
| AEM | 0,06 |
| V50 | 0,018 |

Caractéristiques du sol obtenu:

| | |
|---|---|
| (a) diamètre à 20°C | 610 nm |
| (b) diamètre à 50°C | 450 nm |
| (c) diamètre par MET | 305 nm |
| (d) densité de charge | 19 mmol/g |

| | |
|---|---|
| (a) diamètre mesuré par diffusion dynamique de la lumière à 20°C. | |
| (b) diamètre mesuré par diffusion dynamique de la lumière à 50°C. | |
| (c) diamètre mesuré en Microscopie électronique. | |
| (d) densité de charge exprimée en mmole(amine)/g de polymère. | |

### 2) Les premier et second polymères sont identiques et sont le PNIPAM

Les réactifs de départ sont ceux qui ont été choisis dans 1) pour le second polymère.

### 2.1) Polymérisation en batch (ou procédé en réacteur fermé)

Le monomère (1) (NIPAM), le monomère (3) fonctionnel (AEM) et l'agent de réticulation (MBA) sont introduits ensemble en une seule étape avant que la polymérisation ne soit amorcée par addition de l'amorceur (V50). La durée de polymérisation est de 30 min.

Formulation du polymère obtenu identifié sous la référence PNIPAM42 :

| | |
|---|---|
| Volume total d'eau bouillie et dégazée | 250ml |
| NIPAM | 48,51 mmoles |
| MBA | 3 mmoles |
| AEM | 0,48 mmoles |
| V50 | 0,30 mmoles |
| Température | 70°C |

Les caractéristiques du polymère obtenu après polymérisation sont reportées dans le tableau suivant :

| | |
|---|---|
| diamètre^{(a)} DDL 20°C à 20°C | 292 nm |
| diamètre^{(b)} taille DDL à 40°C | 164 nm |
| diamètre^{(c)} MET | 129 nm |
| concentration en AEM^{(d)} | 14,1 µmole/g de polymère |
| LCST^{(e)} | 31,5°C |
| CCC^{(f)} à 20°C | 1,00 mole/l |

| | |
|---|---|
| (a) diamètre mesuré par diffusion dynamique de la lumière à 20°C | |
| (b) diamètre mesuré par diffusion dynamique de la lumière à 40°C | |
| (c) diamètre mesuré en Microscopie électronique | |
| (d) densité de charge exprimée en mmole(amine primaire)/ g de polymère | |
| (e) température critique inférieure de solubilité (LCST) déterminée par mesure de turbidité en fonction de la température | |
| (f) concentration critique de coagulation (CCC) à 20°C. | |

### 2.2) Polymérisation en semi-continu

Le monomère (3) est introduit en deux étapes, à 3 min et à 6 min respectivement, dans le réacteur renfermant déjà le monomère (1), l'agent de réticulation (2) MBA et l'amorceur V50, en cours de polymérisation. Cet ajout peut être effectué à une vitesse d'injection constante (polymérisation par ajout continu) ou bien suivant un ajout bien contrôlé à des intervalles réguliers (polymérisation en semi-continu). Le but de cette méthode de polymérisation est d'augmenter l'incorporation de monomère(s) (3) fonctionnel(s) sans augmenter le pourcentage de polymère hydrosoluble dans le milieu réactionnel.

Formulation du polymère obtenu identifié sous la référence PNIPAM45 :

| | |
|---|---|
| Volume total d'eau bouillie et dégazée | 250ml |
| NIPAM | 48,51 mmoles |
| MBA | 3 mmoles |
| AEM | 0,48 mmoles |
| V50 | 0,30 mmoles |
| Température | 70°C |
| Ajouts | entre 3 et 6 min |

Les caractéristique du polymère PNIPAM45 obtenu après polymérisation sont reportées dans le tableau suivant :

| | |
|---|---|
| diamètre^{(a)} DDL 20°C à 20°C | 823 nm |
| diamètre^{(b)} taille DDL à 40°C | 530 nm |
| diamètre^{(c)} MET | 327 nm |
| concentration en AEM^{(d)} | 10,0 µmole/g de polymère |
| LCST^{(e)} | 32°C |
| CCC^{(f)} à 20°C | 1,00 mole/l |

| | |
|---|---|
| (a) diamètre mesuré par diffusion dynamique de la lumière à 20°C | |
| (b) diamètre mesuré par diffusion dynamique de la lumière à 40°C | |
| (c) diamètre mesuré en Microscopie électronique | |
| (d) densité de charge exprimée en mmole(amine primaire)/ g de polymère | |
| (e) température critique inférieure de solubilité (LCST) déterminée par mesure de turbidité en fonction de la température | |
| (f) concentration critique de coagulation (CCC) à 20°C. | |

### 2.3) Polymérisation sur semence

Cette méthode consiste à introduire le ou les monomère(s) (1) et/ou (3) dans un milieu réactionnel contenant un sol de polymère préalablement préparé selon 2.1 et parfaitement caractérisé.

Formulation du mélange réactionnel :

Un volume de 40 ml de semence 2.1 à une concentration de 4,5 g pour 100 ml est utilisé. Les réactifs ont été ajoutés dilués dans un volume de 5 ml d'eau. Les pourcentages molaires de NIPAM, de MBA et de V50 ajoutés dans la deuxième étape sont identiques à ceux de la semence selon 2.1. En revanche, la concentration en monomère (3) fonctionnel est contrôlée (augmentée ou diminuée suivant la densité de charge voulue) ; dans le cas présent 10% de AEM sont ajoutés par rapport au monomère (1) NIPAM.

Les caractéristiques du polymère identifié sous la référence PNIPAM94) qui a été obtenu suivant le mode opératoire décrit dans 2.1, sont reportées dans le tableau suivant :

| | |
|---|---|
| diamètre^{(a)} DDL 20°C à 20°C | 504 nm |
| diamètre^{(b)} taille DDL à 40°C | 290 nm |
| diamètre^{(c)} MET | 176 nm |
| concentration en AEM^{(d)} | 22,4 µmole/g de polymère |
| LCST^{(e)} | 32°C |
| CCC^{(f)} à 20°C | 1,10 mole/l |

| | |
|---|---|
| (a) diamètre mesuré par diffusion dynamique de la lumière à 20°C | |
| (b) diamètre mesuré par diffusion dynamique de la lumière à 40°C | |
| (c) diamètre mesuré en Microscopie électronique | |
| (d) densité de charge exprimée en mmole(amine primaire)/ g de polymère | |
| (e) température critique inférieure de solubilité (LCST) déterminée par mesure de turbidité en fonction de la température | |
| (f) concentration critique de coagulation (CCC) à 20°C. | |

### EXEMPLE 2 : SYNTHESE ET CARACTERISATION DES FERROFLUIDES IONIQUES DESTINES A ETRE INCORPORES DANS LA COUCHE DU SECOND POLYMERE

Le mode opératoire a été élaboré selon les résultats énoncés dans le brevet US-4 329 241.

Les propriétés physiques des ferrofluides préparés selon ce document sont rassemblées dans le tableau récapitulatif suivant :

| Propriétés Valeurs Méthodes | | |
|---|---|---|
| diamètres (nm) | 15 ± 3 | FMA (microscopie à force atomique) |
| et | 7 ± 1 | MET (microscopie électronique à transmission) |
| dispersité | 9,5 ± 2 | aimantation (mesure de la magnétisation) |
| | 11 ± 1 | RX (rayons X) |
| épaisseur de la couche non magnétique (nm) | 0,1 | aimantation |
| aimantation spécifique | 422 kA/m | aimantation |
| densité de charge (C/m²) | 1,5 | conductimétrie |
| pH | 7-8 | pH-métrie |
| conductivité | 1 mS | conductimétrie |

La méthode de synthèse par précipitation des oxydes de fer permet, d'après les résultats des différentes méthodes de caractérisation, d'obtenir un ferrofluide anionique, stable entre pH 6 et pH 8, d'une taille de l'ordre d'une dizaine de nm et superparamagnétique. Les analyses ont été effectuées pour différents ferrofluides, obtenus avec le même mode opératoire : les résultats obtenus sont reproductibles d'un ferrofluide à l'autre.

Les particules de ferrofluide étant chargées négativement, il est donc possible de réaliser l'adsorption de ces particules sur un polymère de charge opposée (chargé positivement) via les interactions électrostatiques.

### EXEMPLE 3 : ADSORPTION DE LA CHARGE MAGNETIQUE SUR LA COUCHE DE SECOND POLYMERE

Le second polymère et la charge magnétique (Exemple 2) ont des charges de signe opposé, ce qui favorise une forte adsorption, principalement par interactions électrostatiques, de la charge magnétique sur le polymère. La charge magnétique est placée en excès par rapport à la concentration de polymère (Exemple 1), et l'adsorption est réalisée dans des conditions telles que le taux de recouvrement de la surface du second polymère est supérieur à 30 %. Dans un flacon de 200 ml, 6,4 ml du sol de polymère obtenu à l'Exemple 1 (1.2; concentration = 94,5 g/l) sont progressivement ajoutés à 29 ml de ferrofluide obtenu à l'Exemple 2 (concentration = 23 g/l). Après adsorption de la ferrite pendant 15 minutes, l'excès de ferrofluide est éliminé en plaçant le flacon sur un aimant afin de séparer les particules de polymère recouvertes de ferrite. Le surnageant est éliminé, dosé et remplacé par un même volume d'eau bouillie et dégazée. Le flacon renfermant le polymère recouvert de ferrite est à nouveau placé sur l'aimant afin de vérifier qu'il ne reste plus de ferrite en solution (surnageant limpide).

Le tableau présenté ci-dessus présente la quantité de ferrite adsorbée sur les particules de latex chevelu de l'exemple 1.1 :

| Code | Quantité adsorbée en g/g de latex | % massique adsorbé |
|---|---|---|
| ENC10 | 6,63 | 45 |
| ENC11 | 6,67 | 46 |
| ENC13 | 4,70 | 40 |
| ENC12 | 5,30 | 37 |
| ENC16 | 5,00 | 40 |

### EXEMPLE 4: ENCAPSULATION DES PARTICULES

Le procédé d'encapsulation de la charge magnétique après l'étape d'adsorption consiste à polymériser en milieu aqueux un ou plusieurs monomères avec un agent de réticulation copolymérisable, en présence d'une suspension de polymère magnétique saturée (Exemple 3). Les monomères choisis peuvent être fonctionnels, et dans ce cas, présenter ainsi un intérêt pour le greffage ou l'adsorption des molécules biologiques. Cette méthode permet d'obtenir un polymère magnétique dont l'interface peut facilement être modifiée suivant les utilisations : une surface hydrophobe pour l'adsorption de protéine ou hydrophile fonctionnelle pour un greffage chimique, par exemple.

Le procédé d'encapsulation décrit dans cet exemple repose sur l'utilisation d'un amorceur et d'un mélange de monomères et d'agent de réticulation. 40 ml de polymère recouvert de ferrite (Exemple 3) sont introduits dans un réacteur thermostaté à 70°C. Les monomères sont ensuite introduits dans un volume de 4 ml d'eau bouillie et dégazée. Le temps de polymérisation est de trois heures à compter de l'introduction de l'amorceur. Les monomères utilisés sont indiqués dans les tableaux suivants:
4.1)

| Réactifs | Quantités |
|---|---|
| NIPAM | 0,15 g |
| MBA | 0,0075 g |
| Persulfate de potassium (KPS) | 0,0056 g |

Les particules magnétiques obtenues ont pour références: ENC10, ENC11 et ENC13.
4.2)

| Réactifs | Ouantités |
|---|---|
| NIPAM | 0,15 g |
| MBA | 0,015 g |
| Persulfate de potassium (KPS) | 0,0056 g |

Les particules magnétiques obtenues ont pour références: ENC12 et ENC16.

Ces particules ont été caractérisées. Ces résultats sont présentés dans les tableaux suivants :
a) Diamètre des particules magnétiques obtenues

| code | D^{(a)} nm | Dn^{(b)} nm | Dw^{(b)} nm | IP^{(c)} |
|---|---|---|---|---|
| ENC10 | 500 | 380 | 388 | 1,02 |
| ENC11 | 810 | 385 | 388 | 1,006 |
| ENC13 | 1500 | 352 | 363 | 1,030 |
| ENC12 | 750 | 366 | 369 | 1,007 |
| ENC16 | 1000 | ND | ND | ND |

| | | | | |
|---|---|---|---|---|
| (a) diamètre mesuré par diffusion dynamique de la lumière | | | | |
| (b) diamètre mesuré par microscopie électronique à transmission | | | | |
| (c) indice de polydispersité | | | | |
| ND non déterminé | | | | |

b) Pourcentage massique de ferrite encapsulée dans le polymère et le temps de séparation

| code | % masse ferrite aimantation | % masse ferrite complexométrie | tps de séparation min(*) |
|---|---|---|---|
| ENC10 | 35 | 43 | <20 |
| ENC11 | 23 | 40 | <20 |
| ENC13 | 38 | 36 | <20 |
| ENC12 | 16 | 23 | <30 |
| ENC16 | 40 | 45 | <5 |

| | | | |
|---|---|---|---|
| (*) le temps de séparation est déterminé en utilisant un aimant GENPROBE, Magnetic separation unit catalog#1639, San Diego. | | | |

Les colloïdes obtenus sont stables, monodisperses et présentent un temps de séparation sous l'action d'un champ magnétique inférieur à 30 min, et plus particulièrement inférieur à 5 min (ENC16). Les tailles obtenues sont reproductibles d'une synthèse à l'autre, sont comprises entre 0,1 et 10 µm et la distribution en taille au sein d'une même préparation est très étroite (IP<1,03). La quantité de ferrite encapsulée est comprise entre 40 et 45 %. L'encapsulation ne provoque pas de désorption des particules magnétiques car il a été vérifié que la différence entre le pourcentage massique adsorbé et le pourcentage massique après encapsulation est très faible.

## Revendications

1. Particules superparamagnétiques, monodisperses, ayant une taille prédéterminée comprise entre 0,1 et 10 *µ*m, comprenant :
- un noyau à base d'un premier polymère,
- une couche interne dite couche magnétique, recouvrant le noyau, à base d'un second polymère et dans laquelle est distribué un matériau magnétique, et
- une couche externe, dite couche d'encapsulation, éventuellement fonctionnalisée, recouvrant la couche magnétique, à base d'un troisième polymère et susceptible d'interagir avec au moins une molécule biologique,
**caractérisées en ce que** au moins le second polymère est thermosensible et présente une température critique inférieure de solubilité (LCST) prédéterminée comprise entre 15 et 65 °C et de préférence entre 25 et 50°C.

2. Particules selon la revendication 1, **caractérisées en ce que** le second polymère est obtenu par polymérisation de (1) un monomère hydrosoluble, d'acrylamide ou d'un dérivé d'acrylamide, (2) au moins un agent de réticulation et (3) au moins un monomère fonctionnel, cationique et hydrosoluble, différent du monomère (1).

3. Particules selon la revendication 2, **caractérisées en ce que** le second polymère est le PNIPAM obtenu par polymérisation de (1) N-isopropylacrylamide, (2) de N,N-méthylène bisacrylamide et (3) chlorure de 2-aminoéthyl-méthacrylate.

4. Particules selon la revendication 2 ou 3, **caractérisées en ce que** le premier polymère est identique au second polymère.

5. Particules selon l'une quelconque des revendications 1 à 4, **caractérisées en ce que** le premier polymère est différent du second polymère et est un polymère à caractère hydrophobe, tel qu'un polystyrène ou un polyméthacrylate de méthyle.

6. Particules selon l'une quelconque des revendications 1 à 5, **caractérisées en ce que** le troisième polymère est un polymère compatible avec le second polymère et est choisi parmi les polymères hydrophiles, en particulier les dérivés acrylamides et de préférence le PNIPAM.

7. Particules selon la revendication 6, **caractérisées en ce que** le troisième polymère est fonctionnalisé et porte un groupe fonctionnel choisi parmi les fonctions carboxylique, aldéhydique, thiol et amine.

8. Particules selon l'une quelconque des revendications précédentes, **caractérisées en ce qu**'elles sont de forme essentiellement sphérique.

9. Procédé d'obtention de particules telles que définies selon l'une quelconque des revendications précédentes, **caractérisé en ce que** :
- selon une étape (a) dite étape d'obtention du premier polymère, on obtient par polymérisation du ou des monomères appropriés, le premier polymère,
- selon une étape (b) dite étape d'obtention du second polymère, on obtient un sol du second polymère, par polymérisation en phase aqueuse de (1) un monomère hydrosoluble, d'acrylamide ou d'un dérivé d'acrylamide, (2) au moins un agent de réticulation et (3) au moins un monomère fonctionnel, cationique et hydrosoluble, différent du monomère (1),
- selon une étape (c) dite d'adsorption du matériau magnétique, on met en contact le matériau magnétique avec les premier et second polymères, à une température inférieure à la LCST du second polymère,
- selon une étape (d) dite d'obtention de la couche magnétique, on porte le mélange réactionnel obtenu selon (c) à une température supérieure à la LCST du second polymère,
- selon une étape (e) dite d'encapsulation, on met en contact, en phase aqueuse, le mélange obtenu selon (d) avec le ou les monomères appropriés pour l'obtention par polymérisation du troisième polymère.

10. Procédé selon la revendication 9, **caractérisé en ce qu**'on effectue simultanément les étapes (a) et (b).

11. Procédé selon la revendication 10, caractérisé en ce le premier polymère est identique au second polymère.

12. Procédé selon la revendication 9 ou 10, **caractérisé en ce que** le premier polymère est différent du second polymère et est un polymère à caractère hydrophobe, tel qu'un polystyrène ou un polyméthacrylate de méthyle.

13. Procédé selon l'une quelconque des revendications 9 à 12, **caractérisé en ce que** pour l'étape (b) et éventuellement l'étape (a), le monomère (1) est choisi parmi les N-alkylacrylamides et les N,N-dialkylacrylamides.

14. Procédé selon la revendication 13, **caractérisé en ce que** le monomère (1) est choisi parmi le N-isopropylacrylamide, le N-éthylméthacrylamide, N-n-propylacrylamide, le N-n-propylméthacrylamide, N-isopropylméthacrylamide, le N-cyclopropylacrylamide, le N,N-diéthylacrylamide, le N-méthyl-N-isopropylacrylamide, le N-méthyl-N-n-propylacrylamide, le monomère (1) étant de préférence le N-isopropylacrylamide (NIPAM).

15. Procédé selon l'une quelconque des revendications 9 à 14, **caractérisé en ce que** pour l'étape (b) et éventuellement l'étape (a), le ou les monomères fonctionnels (3) sont choisis parmi les dérivés acryliques et méthacryliques, le chlorure de 2-aminoéthyl-méthacrylate (AEM), les dérivés de N-vinyl-pyridine, les dérivés de trialkylammonium et les dérivés de chlorure d'isothiouronium.

16. Procédé selon les revendications 9 ou 10 et 11, **caractérisé en ce que** pour l'étape (b) et éventuellement l'étape (a), l'agent de réticulation (2) est hydrosoluble et est choisi parmi le N,N-méthylène bisacrylamide (MBA), l'éthylène glycol diméthacrylate.

17. Procédé selon l'une quelconque des revendications 9 à 16, **caractérisé en ce que** le troisième polymère est choisi parmi les polymères hydrophiles, en particulier les dérivés acrylamides et de préférence le PNIPAM.

18. Utilisation de particules telles que définies selon l'une quelconque des revendications 1 à 8, pour isoler au moins une molécule biologique, notamment choisie parmi les protéines, les anticorps, les fragments d'anticorps, les antigènes, les polypeptides, les enzymes, les haptènes, les acides nucléiques et les fragments d'acides nucléiques.

19. Utilisation selon la revendication 18, selon laquelle on fixe sur les particules, par adsorption ou par liaison covalente, directement ou indirectement, la ou les molécules biologiques.

20. Procédé pour isoler, dans un échantillon liquide, au moins une molécule biologique, selon lequel :
- on dispose de particules selon l'une quelconque des revendications 1 à 8,
- on met en contact ledit échantillon avec lesdites particules, par incubation,
- on applique un champ magnétique au mélange obtenu,
- on sépare les particules de l'échantillon.

21. Réactif pour l'isolement de molécules biologiques, **caractérisé en ce qu**'il comprend une dispersion en milieu aqueux de particules telles que définies selon l'une quelconque des revendications 1 à 8.

## Patentansprüche

1. Monodisperse superparamagnetische Partikel, die eine festgelegte Größe von 0,1 bis 10 *µ*m aufweisen mit
- einem Kern auf Basis eines ersten Polymers,
- einer inneren Schicht, magnetische Schicht genannt, die den Kern bedeckt, auf Basis eines zweiten Polymers, und in der ein magnetisches Material verteilt ist, und
- einer äußeren Schicht, ggf. funktionalisiert, Verkapselungsschicht genannt, die die magnetische Schicht bedeckt, auf Basis eines dritten Polymers, und welche geeignet ist, mit zumindest einem biologischen Molekül zu interagieren,
**dadurch gekennzeichnet, daß** zumindest das zweite Polymer wärmeempfindlich ist und eine bestimmte untere kritische Lösungstemperatur (LCST) aufweist, die bei 15 bis 65°C, und vorzugsweise bei 25 bis 50°C liegt.

2. Partikel nach Anspruch 1, **dadurch gekennzeichnet**, daß das zweite Polymer durch Polymerisation von (1) einem wasserlöslichen Monomer eines Acrylamides oder eines Acrylamidderivates, (2) zumindest einem Vernetzungsagens und (3) zumindest einem kationischen und wasserlöslichen funktionellen Monomer, das sich von dem Monomer (1) unterscheidet, erhalten wird.

3. Partikel nach Anspruch 2, **dadurch gekennzeichnet**, daß das zweite Polymer PNIPAM ist, das durch Polymerisation von (1) N-Isopropylacrylamid, (2) N,N-Methylenbisacrylamid und (3) 2-Aminoethylmethacrylchlorid erhalten wird.

4. Partikel nach Anspruch 2 oder 3, **dadurch gekennzeichnet**, daß das erste Polymer mit dem zweiten Polymer identisch ist.

5. Partikel nach einem der Ansprüche 1 bis 4, **dadurch gekennzeichnet**, daß sich das erste Polymer von dem zweiten Polymer unterscheidet und ein Polymer mit hydrophober Eigenschaft, wie z.B. ein Polystyrol oder ein Methylpolymethacrylat, ist.

6. Partikel nach einem der Ansprüche 1 bis 5, **dadurch gekennzeichnet**, daß das dritte Polymer mit dem zweiten Polymer kompatibel ist und ausgewählt ist aus den hydrophilen Polymeren, insbesondere den Acrylamidderivaten, und vorzugsweise PNIPAM ist.

7. Partikel nach Anspruch 6, **dadurch gekennzeichnet**, daß das dritte Polymer funktionalisiert ist und eine funktionelle Gruppe trägt, ausgewählt aus Carboxyl-, Aldehyd-, Thiol- und Amingruppen.

8. Partikel nach einem der vorhergehenden Ansprüche, **dadurch gekennzeichnet**, daß sie im wesentlichen kugelförmig sind.

9. Verfahren zum Herstellen der nach einem der vorhergehenden Ansprüche definierten Partikel, **dadurch gekennzeichnet**, daß:
- gemäß eines Schrittes (a), Schritt zum Herstellen des ersten Polymers genannt, das erste Polymer durch Polymerisation des oder der geeigneten Monomere erhalten wird,
- gemäß eines Schrittes (b), Schritt zum Herstellen des zweiten Polymers genannt, ein Sol des zweiten Polymers durch Polymerisation in wässriger Phase von (1) einem wasserlöslichen Monomer von Acrylamid oder von einem Acrylamidderivat, (2) zumindest einem Vernetzungsagens und (3) zumindest einem kationischen und wasserlöslichen funktionellen Monomer, das sich von dem Monomer (1) unterscheidet, erhalten wird,
- gemäß eines Schrittes (c), Adsorption des magnetischen Materials genannt, das magnetische Material bei einer Temperatur, die unterhalb der LCST des zweiten Polymers liegt, mit dem ersten und zweiten Polymer in Kontakt gebracht wird,
- gemäß eines Schrittes (d), Herstellen der Magnetschicht genannt, das gemäß (c) erhaltene reaktive Gemenge, auf eine Temperatur gebracht wird, die über der LCST des zweiten Polymers liegt, und
- gemäß eines Schrittes (e), Verkapselung genannt, das gemäß (d) erhaltene Gemenge in wässriger Phase mit dem oder den geeigneten Monomeren in Kontakt gebracht wird, um durch Polymerisation das dritte Polymer zu erhalten.

10. Verfahren nach Anspruch 9, **dadurch gekennzeichnet**, daß die Schritte (a) und (b) gleichzeitig durchgeführt werden.

11. Verfahren nach Anspruch 10, **dadurch gekennzeichnet**, daß das erste Polymer mit dem zweiten Polymer identisch ist.

12. Verfahren nach Anspruch 9 oder 10, **dadurch gekennzeichnet**, daß sich das erste Polymer von dem zweiten Polymer unterscheidet und ein Polymer mit hydrophober Eigenschaft, wie z.B. ein Polystyrol oder ein Methylpolymethacrylat, ist.

13. Verfahren nach einem der Ansprüche 9 bis 12, **dadurch gekennzeichnet**, daß für den Schritt (b) und ggf. für den Schritt (a) das Monomer (1) ausgewählt ist aus N-Alkylacrylamiden und N,N-Dialkylacrylamiden.

14. Verfahren nach Anspruch 13, **dadurch gekennzeichnet**, daß das Monomer (1) ausgewählt ist aus N-Isopropylacrylamid, N-Ethylmethacrylamid, N-n-Propylacrylamid, N-n-Propylmethacrylamid, N-Isopropylmethacrylamid, N-Cyclopropylacrylamid, N,N-Diethylacrylamid, N-Methyl-N-isopropylacrylamid, N-Methyl-N-n-propylacrylamid, wobei das Monomer (1) vorzugsweise N-Isopropylacrylamid (NIPAM) ist.

15. Verfahren nach einem der Ansprüche 9 bis 14, **dadurch gekennzeichnet**, daß für den Schritt (b) und ggf. für den Schritt (a) das oder die funktionellen Monomere (3) ausgewählt sind aus den Acryl- und Methacrylderivaten, 2-Aminoethylmethacrylat(AEM)-Chlorid, den N-Vinylpyridinderivaten, den Trialkylammoniumderivaten und den Isothiouroniumchloridderivaten.

16. Verfahren nach den Ansprüchen 9 oder 10 oder 11, **dadurch gekennzeichnet**, daß für den Schritt (b) und ggf. für den Schritt (a) das Vernetzungsagens (2) wasserlöslich ist und ausgewählt ist aus N,N-Methylenbisacrylamid (MBA), Ethylenglykoldimethacrylat.

17. Verfahren nach einem der Ansprüche 9 bis 16, **dadurch gekennzeichnet**, daß das dritte Polymer ausgewählt ist aus den hydrophilen Polymeren, insbesondere den Acrylamidderivaten, und vorzugsweise PNIPAM ist:.

18. Verwendung der nach einem der Ansprüche 1 bis 8 definierten Partikel, um zumindest ein biologisches Molekül zu isolieren, insbesondere ausgewählt aus den Proteinen, den Antikörpern, den Antikörperfragmenten, den Antigenen, den Polypeptiden, den Enzymen, den Haptenen, den Nukleinsäuren und Nukleinsäurefragmenten.

19. Verwendung nach Anspruch 18, wobei das oder die biologischen Moleküle direkt oder indirekt durch Adsorption oder durch kovalente Bindung auf die Partikel fixiert werden.

20. Verfahren, um in einer flüssigen Probe zumindest ein biologisches Molekül zu isolieren, nachdem:
- Partikel nach einem der Ansprüche 1 bis 8 bereitgestellt werden,
- die Probe durch Inkubation mit den Partikeln in Kontakt gebracht wird,
- an das erhaltene Gemenge ein Magnetfeld angelegt wird, und
- die Partikel von der Probe abgetrennt werden.

21. Reagens zum Isolieren von biologischen Molekülen, **dadurch gekennzeichnet**, daß es eine Dispersion von nach einem der Ansprüche 1 bis 8 definierten Partikeln in wässrigem Milieu aufweist.

## Claims

1. Monodisperse superparamagnetic particles having a predetermined size of between 0.1 and 10 µm, comprising:
- a core based on a first polymer,
- an internal layer, called the magnetic layer, covering the core, based on a second polymer, in which layer a magnetic material is distributed, and
- an external layer, called the encapsulation layer, optionally functionalized, covering the magnetic layer, based on a third polymer and capable of interacting with at least one biological molecule,
**characterized in that** at least the second polymer is heat-sensitive and has a predetermined lower critical solubility temperature (LCST) of between 15 and 65°C and preferably between 25 and 50°C.

2. Particles according to Claim 1, **characterized in that** the second polymer is obtained by polymerization of (1) a water-soluble monomer of acrylamide or of an acrylamide derivative, (2) at least one crosslinking agent and (3) at least one functional, cationic and water-soluble monomer different from the monomer (1).

3. Particles according to Claim 2, **characterized in that** the second polymer is PNIPAM obtained by polymerization of (1) N-isopropylacrylamide, (2) of N,N-methylenebisacrylamide and (3) 2-aminoethylmethacrylate chloride.

4. Particles according to Claim 2 or 3, **characterized in that** the first polymer is identical to the second polymer.

5. Particles according to any one of Claims 1 to 4, **characterized in that** the first polymer is different from the second polymer and is a polymer having a hydrophobic character, such as a polystyrene or a polymethyl methacrylate.

6. Particles according to any one of Claims 1 to 5, **characterized in that** the third polymer is a polymer compatible with the second polymer and is selected from hydrophilic polymers, in particular acrylamide derivatives and preferably PNIPAM.

7. Particles according to Claim 6, **characterized in that** the third polymer is functionalized and bears a functional group selected from carboxylic, aldehyde, thiol and amine functional groups.

8. Particles according to any one of the preceding claims, **characterized in that** they have an essentially spherical shape.

9. Process for obtaining particles as defined according to any one of the preceding claims, **characterized in that**:
- in a step (a), called the step for obtaining the first polymer, the first polymer is obtained by polymerization of the suitable monomer or monomers,
- in a step (b), called the step for obtaining the second polymer, a sol of the second polymer is obtained by polymerization in aqueous phase of (1) a water-soluble monomer of acrylamide or of an acrylamide derivative, (2) at least one crosslinking agent and (3) at least one functional, cationic and water-soluble monomer different from the monomer (1),
- in a step (c), called the step for adsorption of the magnetic material, the magnetic material is brought into contact with the first and second polymers at a temperature below the LCST of the second polymer,
- in a step (d), called the step for obtaining the magnetic layer, the reaction mixture obtained in (c) is raised to a temperature above the LCST of the second polymer, and
- in a step (e), called the encapsulation step, the mixture obtained in (d) is brought into contact, in aqueous phase, with the monomer or monomers suitable for obtaining the third polymer by polymerization.

10. Process according to Claim 9, **characterized in that** steps (a) and (b) are carried out simultaneously.

11. Process according to Claim 10, **characterized in that** the first polymer is identical to the second polymer.

12. Process according to Claim 9 or 10, **characterized in that** the first polymer is different from the second polymer and is a polymer having a hydrophobic character, such as a polystyrene or a polymethyl methacrylate.

13. Process according to any one of Claims 9 to 12, **characterized in that**, for step (b) and optionally step (a) the monomer (1) is selected from N-alkylacrylamides and N,N-dialkylacrylamides.

14. Process according to Claim 13, **characterized in that** the monomer (1) is selected from N-isopropylacrylamide, N-ethylmethacrylamide, N-n-propylacrylamide, N-n-propylmethacrylamide, N-isopropylmethacrylamide, N-cyclopropylacrylamide, N,N-diethylacrylamide, N-methyl-N-isopropylacrylamide, N-methyl-N-n-propylacrylamide, the monomer (1) preferably being N-isopropylacrylamide (NIPAM).

15. Process according to any one of Claims 9 to 14, **characterized in that**, for step (b) and optionally step (a), the functional monomer or monomers (3) are selected from acrylic and methacrylic derivatives, 2-aminoethylmethacrylate (AEM) chloride, N-vinylpyridine derivatives, trialkylammonium derivatives and isothiouronium chloride derivatives.

16. Process according to Claims 9 or 10 and 11, **characterized in that**, for step (b) and optionally step (a), the crosslinking agent (2) is water-soluble and is selected from N,N-methylenebisacrylamide (MBA) and ethylene glycol dimethacrylate.

17. Process according to any one of Claims 9 to 16, **characterized in that** the third polymer is selected from hydrophilic polymers, in particular acrylamide derivatives and preferably PNIPAM.

18. Use of particles as defined in any one of Claims 1 to 8, in order to isolate at least one biological molecule, in particular selected from proteins, antibodies, fragments of antibodies, antigens, polypeptides, enzymes, haptens, nucleic acids and fragments of nucleic acids.

19. Use according to Claim 18, in which the biological molecule or molecules are fixed to the particles, directly or indirectly, by adsorption or by covalent bonding.

20. Process for isolating, in a liquid specimen, at least one biological molecule, in which:
- particles according to any one of Claims 1 to 8 are used,
- said specimen is brought into contact with said particles, by incubation,
- a magnetic field is applied to the mixture obtained and
- the particles are separated from the specimen.

21. Reactive means for the isolation of biological molecules, **characterized in that** it comprises a dispersion, in aqueous medium, of particles as defined in any one of Claims 1 to 8.
